# EUROPÄISCHE PATENTSCHRIFT

(11) **EP 0 600 199 B1**
(45) Veröffentlichungstag und Bekanntmachung des Hinweises auf die Patenterteilung: **23.04.1997**
(21) Anmeldenummer: 93116653.2
(22) Anmeldetag: 14.10.1993
(51) Int. Cl.: A61G 13/02

(54) **Untersuchungstisch mit einer Lagerungsplatte und einem Bodenstativ, welches in der Längsrichtung der Lagerungsplatte verschiebbar ist**
Examining table including a support area and a stand, the support area is moveable in its longitudinal direction
Table d'examination comprenant une surface de support et un socle, la surface de support est mobile selon son axe longitudinal

(30) Priorität: 03.12.1992 SE 9203643
(43) Veröffentlichungstag der Anmeldung: 08.06.1994
(73) Patentinhaber: Siemens-Elema AB, 171 95 Solna 1 (SE)
(72) Erfinder: Barud, Sigvard, S-175 70 Järfälla (SE)

(56) Entgegenhaltungen:
- EP-A- 0 009 463
- US-A- 3 843 112

## Beschreibung

Die Erfindung betrifft einen Untersuchungstisch für einen Patienten mit einer Lagerungsplatte und einem Bodenstativ, wobei der Untersuchungstisch einen Gelenkarm aufweist, der zwischen dem Bodenstativ und der Lagerungsplatte angeordnet ist, mit dem die Lagerungsplatte gegenüber dem Bodenstativ in der Längsrichtung der Lagerungsplatte verschiebbar ist.

Bei Röntgenuntersuchungen, z.B. in Verbindung mit Katheterisierungen, ist es von Bedeutung, daß die Röntgenröhre und der Bildschichtträger eines Röntgenuntersuchungsstatives einen guten Zugang zum Patienten erhält. Bei derartigen Untersuchungen ist in der Regel der Untersuchungstisch und das Röntgenuntersuchungsstativ parallel zueinander verschiebbar, so dass z.B. die Röntgenröhre und der Bildschichtträger vom Kopfende bis zum Fussende des Untersuchungstisches verschoben werden kann, wobei der Bildschichtträger bei einer frontalen Projektion auch über die gesamte Länge unterhalb der Lagerungsplatte freien Raum haben muss.

Ein Tisch der eingangs genannten Art, ist durch die US-A-3,843,112 bekannt. Dieser Tisch, der für Operationen vorgesehen ist, weist als Gelenkarm einen einzigen steifen Arm auf, der einerseits mit der Lagerungsplatte und andererseits mit einer als Bodenstativ dienenden Bedienungskonsole drehbar verbunden ist. Beim Verschieben der Lagerungsplatte in ihre Längsrichtung erfolgt eine halbkreisförmige Schwenkung der Lagerungsplatte um die Befestigungsstelle des Armes an der Bedienungskonsole. Eine solche Verschiebung der Lagerungsplatte ist insbesondere in Verbindung mit Katheterisierungen und Einspritzen von Kontrastmitteln in den Patienten sehr ungünstig, da der Weg des Katheters bzw. der Kontrastmittel mit Hilfe eines Röntgenuntersuchungsgerätes schwer zu verfolgen ist. Der Operationstisch weist ferner einen weiteren Arm auf, der einerseits am Boden und andererseits an der Bedienungskonsole drehbar befestigt ist. Durch diesen zusätzlichen Arm kann die Lagerungsplatte und die Bedienungskonsole um das bodenfeste Ende des Armes von einem Raum in einen anderen geschwenkt werden. Eine Parallelverschiebung der Lagerungsplatte in ihrer Längsrichtung gegenüber der Bedienungskonsole ist auch bei dieser Schwenkung nicht gegeben. Da die Lagerungsplatte bei einer Verschiebung den Bereich der Bedienungskonsole in keiner der beschriebenen Lagen richtig verläßt, ist auch kein guter Zugang für ein Röntgenuntersuchungsstativ gegeben.

Ein Untersuchungstisch, der für Katheterisierungsuntersuchungen geeignet ist, ist durch den Siemens-Prospekt "KOORDINAT ANGIO" bekannt. Die Lagerungsplatte ruht auf einem festen Bodenstativ und kann mittels eines teleskopähnlichen Schienensystems, das am Bodenstativ befestigt ist, verschoben werden, so dass die gesamte Länge der Lagerungssplatte vom Bodenstativ herausragt. Das Bodenstativ ist dazu vorgesehen, teils einen Filmwechsler und teils Anordnungen zum Kippen der Lagerungsplatte aufzunehmen und ist daher zusammenfassend verhältnismässig gross, im Aufbau kompliziert und dadurch in der Herstellung teuer.

Ein weiterer Untersuchungstisch, der für das Röntgenuntersuchungsstativ freien Raum bietet, ist durch die US-PS 5 013 018 bekannt. Die Lagerungsplatte ist an ihrer einen Längsseite mit einem säulenförmigen Bodenstativ fest verbunden. Die Verschiebung der Lagerungsplatte in ihrer Längsrichtung erfolgt mit Hilfe von Bodenschienen. Bodenschienen werden immer als ein Hindernis in einem Untersuchungsraum betrachtet. Ausserdem ist es immer schwer, sie klinisch sauber zu halten.

In der EP-A-0 009 463 ist ein wandfester Tisch oder eine Halterung beschrieben. Mit Hilfe eines zweiteiligen Knickarmes, der die Wandbefestigung mit dem Tisch verbindet, kann der Tisch in verschiedene Lagen gebracht werden. Die beschriebene Anordnung ist für ein Fernsehgerät vorgesehen.

Der Erfindung liegt die Aufgabe zugrunde, einen Untersuchungstisch der eingangs genannten Art zu schaffen, der im Aufbau verhältnismässig einfach ist und der einen sehr guten Zugang für ein Röntgenuntersuchungsstativ erlaubt.

Diese Aufgabe ist erfindungsgemäss dadurch gelöst, dass der Gelenkarm mindestens zwei Arme aufweist, deren eine Enden um eine erste Welle miteinander drehbar verbunden sind, und daß das andere Ende des ersten Armes mit der Lagerungsplatte um eine zweite Welle drehbar verbunden und das andere Ende des zweiten Armes um eine dritte Welle mit dem Bodenstativ drehbar verbunden ist, wobei die Wellen an den Verbindungsstellen gegenüber der horizontalen Ebene der Lagerungsplatte vertikal angeordnet sind, so dass sich die Arme immer parallel zu dieser Ebene bewegen, wobei der Gelenkarm mit Hilfe von Mitteln derart steuerbar ist, dass der Winkel zwischen der Längsseite der Lagerungsplatte und dem ersten Arm in allen Lagen gleich dem Winkel zwischen der Längsseite der Lagerungsplatte und dem zweiten Arm ist. Indem die Lagerungsplatte nicht direkt auf dem Bodenstativ ruht, kann das Bodenstativ ziemlich klein gehalten werden. Ausserdem kann die Lagerungsplatte durch den zweiteiligen Gelenkarm eine im Vergleich zu anderen bekannten Untersuchungstischen sehr lange translatorische Bewegung ausführen.

In einer vorteilhaften Ausbildung der Erfindung wird vorgeschlagen, dass die Arme des Gelenkarmes in verschiedenen Ebenen angebracht sind. Auf diese Weise kann der Gelenkarm, ausgehend von dessem Bodenbefestigungspunkt, in alle Richtungen gleich weit ausgestreckt werden.

In einer Weiterbildung der Erfindung wird vorgeschlagen, dass das andere Ende des ersten Armes mit dem einen Ende der Lagerungsplatte, vorzugsweise mit dem Fussende, verbunden ist. Hierdurch wird erreicht, dass teils keine für eine Röntgenuntersuchung störenden Teile unterhalb der Lagerungsplatte plaziert sind und teils, dass die Lagerungsplatte maximal weit vom Bodenstativ herausragen kann.

In einer vorteilhaften Ausgestaltung der Erfindung wird vorgeschlagen, dass die Länge der Arme die grösste Breite der Lagerungsplatte nicht überschreitet. Hierdurch ist sichergestellt, dass die Arme bei einer Verschiebung der Lagerungsplatte nicht über die Längsseite der Lagerungsplatte herausragen.

Im Hinblick auf eine weitere Ausbildung der Erfindung wird vorgeschlagen, dass die Steuerung des Gelenkarmes mittels dreier Motoren erfolgt, bei denen der erste Motor den zweiten Arm um die dritte Welle dreht, der zweite Motor den ersten Arm um die erste Welle dreht, und der dritte Motor die Lagerungsplatte um die zweite Welle dreht. Hierdurch wird erreicht, dass die Lagerungsplatte in eine gewünschte Lage automatisch verschiebbar ist. Die Motoren können vorzugsweise Schrittmotoren sein, die die Lagerungsplatte schrittweise in ihre Längsrichtung verschieben. Solche Bewegungen sind bei Röntgenuntersuchungen insbesondere in Verbindung mit einer Beinkatheterisierung von Vorteil.

Eine vorteilhafte Weiterbildung der Erfindung ergibt sich daraus, dass der Gelenkarm ein am Bodenstativ fest angebrachtes erstes Zahnrad, das um die dritte Welle angeordnet und im zweiten Arm angebracht ist, und ein zweites Zahnrad, das im zweiten Arm angeordnet ist, um die erste Welle drehbar und mit dem ersten Arm fest verbunden ist, sowie eine Transmission, die zwischen dem ersten und dem zweiten Zahnrad angeordnet ist, umfasst, wobei das erste und das zweite Zahnrad derart dimensioniert sind, dass eine Übersetzung 2:1 erreicht wird, wobei der Gelenkarm ferner ein drittes Zahnrad, das im ersten Arm angebracht und um die erste Welle angeordnet und mit dem zweiten Arm fest verbunden ist, und ein viertes Zahnrad, das im ersten Arm appliziert und um die zweite Welle drehbar und mit der Lagerungsplatte fest verbunden ist, umfasst, wobei eine Transmission zwischen dem dritten und dem vierten Zahnrad mit einer Übersetzung 1:2 eingestellt ist. Durch diesen Aufbau des Gelenkarmes wird bei einer Verschiebung der Lagerungsplatte diese dazu gezwungen, eine translatorische Bewegung in ihrer Längsrichtung vorzunehmen. Eine solche Verschiebung der Lagerungsplatte soll manuell ausgeführt werden können. Der zweite Arm kann auch mittels eines Motors, z.B. eines Schrittmotors gedreht werden, so dass auch bei dieser Ausführungsform des Gelenkarmes eine automatische Verschiebung der Lagerungsplatte möglich ist.

Weitere Vorteile und Einzelheiten ergeben sich aus den Unteransprüchen.

Die Erfindung ist nachfolgend anhand eines in den Zeichnungen dargestellten Ausführungsbeispiels näher erläutert. Es zeigen:
- FIG 1: eine Seitenansicht eines Untersuchungstisches nach der Erfindung
- FIG 2 und 3: Draufsichten eines Untersuchungstisches nach FIG 1,
- FIG 4: eine Seitenansicht eines Gelenkarmes für einen Untersuchungstisch nach der Erfindung, bei der der Gelenkarm im Schnitt gezeigt ist, und
- FIG 5: einen schematisch dargestellten Gelenkarm nach der FIG. 4 in einer Draufsicht.

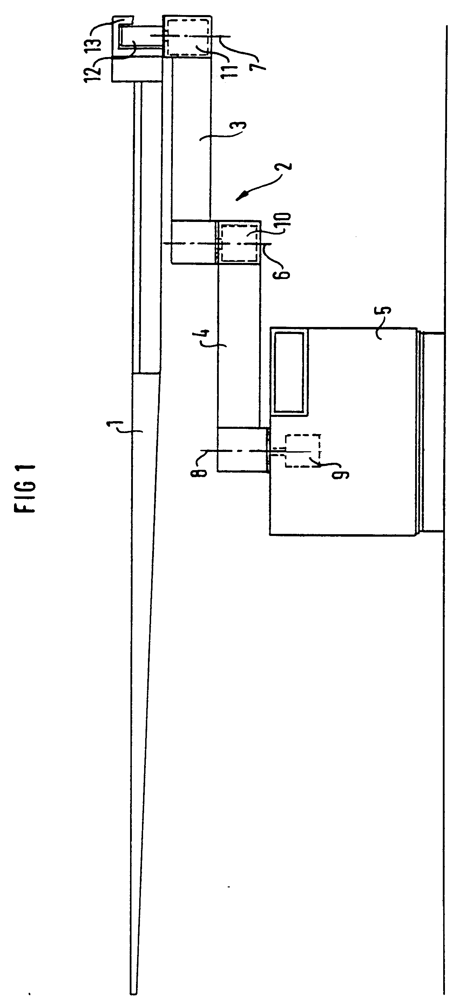

In der FIG. 1 ist ein Untersuchungstisch mit einer Lagerungsplatte 1 dargestellt, die über einen Gelenkarm 2, bestehend aus einem ersten und einem zweiten Arm 3 und 4 mit einem in der Höhe einstellbaren Bodenstativ 5 verbunden ist. Das eine Ende des ersten Armes 3 und das andere Ende des zweiten Armes 4 sind um eine erste Welle 6 miteinander drehbar verbunden, wobei das andere Ende des ersten Armes 3 mit dem Fussende der Lagerungsplatte 1 um eine zweite Welle 7 drehbar verbunden und das andere Ende des zweiten Armes 4 mit dem Bodenstativ 5 um eine dritte Welle 8 drehbar verbunden ist. Das Ende des Armes 3 an der Welle 7 ist mit einem nach oben ragenden Lager 12 versehen, das mit einer am Fussende der Lagerungsplatte 1 fest angebrachten Schiene 13, die auf dem Lager 12 verschiebbar ist, verbunden ist. Die Wellen 6, 7 und 8 sind gegenüber der horizontalen Ebene der Lagerungsplatte 1 vertikal angeordnet, so dass die Arme 3, 4 sich immer parallel mit dieser Ebene bewegen.

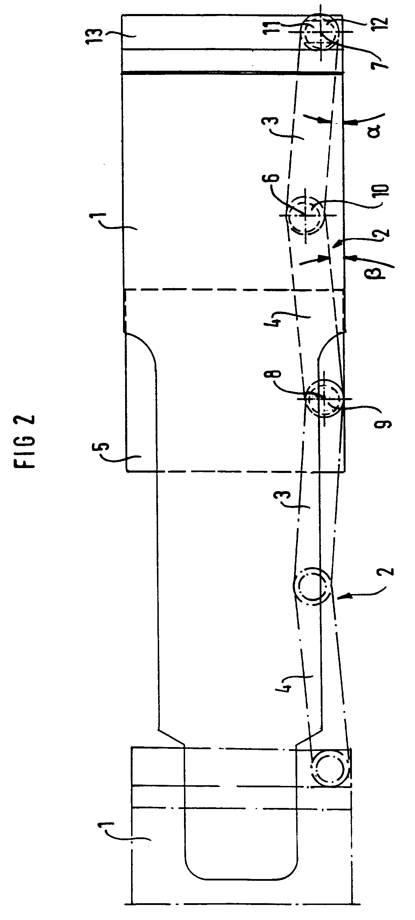

Die Steuerung des Gelenkarmes 2 und damit auch die Steuerung der Lagerungsplatte 1 erfolgt in diesem Ausführungsbeispiel mittels drei Motoren 9, 10, 11. Der erste Motor 9, der im Bodenstativ 5 angebracht ist, kann den zweiten Arm 4 um die Welle 8 drehen. Der Motor 10 ist im zweiten Arm 4 angeordnet und kann den ersten Arm 3 um die Welle 6 drehen, wobei der Motor 11, der im ersten Arm 3 placiert ist, die Lagerungsplatte 1 um die Welle 7 drehen kann. Die Motoren 9, 10 und 11 können nun den Gelenkarm 2 derart steuern, dass der Winkel α zwischen der Längsseite 1 der Lagerungsplatte und dem ersten Arm 3 in allen Lagen gleich dem Winkel β zwischen der Längsseite der Lagerungsplatte 1 und dem zweiten Arm 4 ist (FIG. 2). Damit ist auch sichergestellt, dass, wenn der Motor 9 den zweiten Arm 4 um die Welle 8 dreht, die Lagerungsplatte 1 in ihre Längsrichtung verschoben wird. Vorrichtungen zur Steuerung von Motoren wie es in diesem Ausführungsbeispiel beschrieben ist, sind durch die Robottechnik bekannt und werden daher nicht in der Anmeldung näher angegeben. Die in der FIG 2 strichpunktierte Version des Gelenkarmes 2 und der Lagerungsplatte 1 soll eine äusserste Lage, in die die Lagerungsplatte 1 verschoben werden kann, darstellen. In dieser FIG. wird auch gezeigt, dass sich die Schiene 13 über die ganze Schmalseite der Lagerungsplatte 1 erstreckt. Auf diese Weise kann die Lagerungsplatte 1 auf dem Lager 12 eine Strecke senkrecht zur Längsrichtung der Lagerungsplatte 1 verschoben werden, die etwa der Breite der Lagerungsplatte 1 entspricht. Eine solche Verschiebung kann manuell vorgenommen werden, wobei die Lagerungsplatte 1 mit Hilfe von bekannten und daher nicht dargestellten Mitteln in einer gewünschten Seitenlage arretiert werden kann.

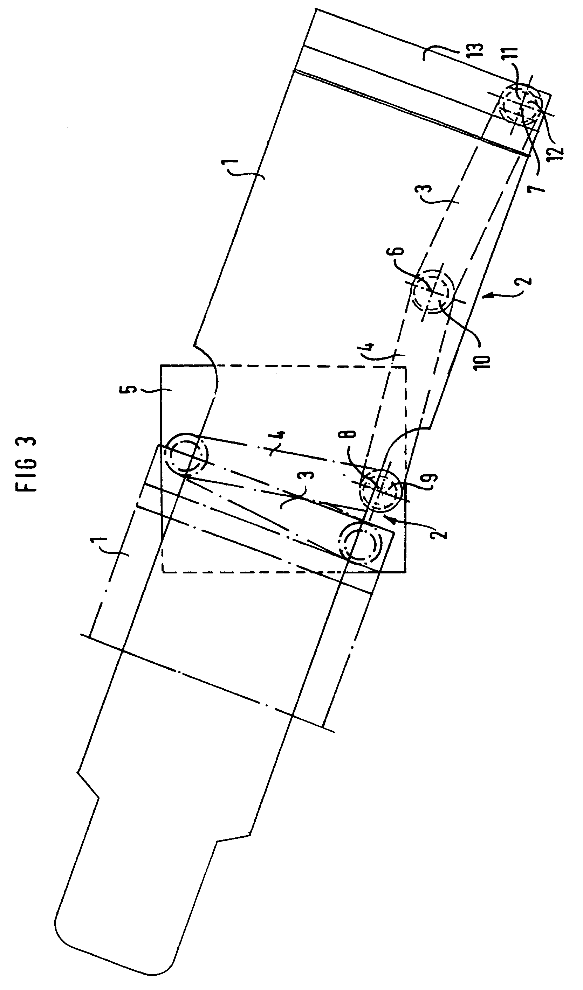

In der FIG.3 ist gezeigt, dass die Lagerungsplatte 1 um die Welle 8 in eine gegenüber dem Bodenstativ 5 gewünschten Lage gedreht werden kann. Von dieser neu eingestellten Lage kann die Lagerungsplatte 1, wie bereits beschrieben, in ihre Längsrichtung verschoben werden. Da die Länge der Arme 3 und 4 nicht die grösste Breite der Lagerungsplatte überschreitet, werden die Arme 3, 4 auch nicht dann über die Lagerungsplatte 1 herausragen, wenn diese eine Lage einnehmen, die senkrecht zur Längsseite der Lagerungsplatte 1 ist. Die strichpunktierte Version des Gelenkarmes 2 und der Lagerungsplatte 1 in dieser FIG 3 zeigt, dass die Lagerungsplatte 1 in eine Lage verschoben ist, in der die Arme 3, 4 eine derartige, gegenüber der Längsseite der Lagerungsplatte 1 fast senkrechte Position eingenommen haben.

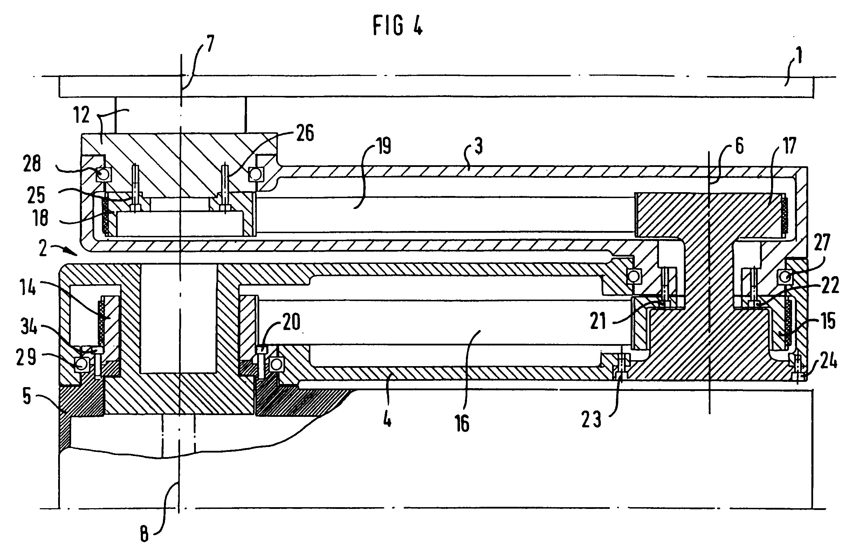

In einem in der FIG.4 dargestellten Ausführungsbeispiel wird der Gelenkarm 2 von einer Riemen- oder Kettentransmission, die die Motoren 9,10, 11 ersetzen, gesteuert. Der Gelenkarm 2 umfasst ein am Bodenstativ 5 fest angebrachtes Zahnrad 14, das um die Welle 8 angeordnet und im Arm placiert ist. Im Arm 4 ist auch ein zweites Zahnrad 15 placiert, das um die Welle 6 drehbar und mit dem Arm 3 fest verbunden ist. Zwischen den Zahnrädern 14 und 15 ist ein Riemen oder eine Kette 16 angeordnet. Die Zahnräder 14 und 15 sind auch derart dimensioniert, dass eine Übersetzung 2:1 erreicht wird. Der Gelenkarm 2 umfasst ausserdem ein drittes Zahnrad 17, das im Arm 3 placiert und um die Welle 8 angeordnet und mit dem Arm 4 fest verbunden ist. Auch ein viertes Zahnrad 18 ist im Arm 3 placiert. Der Arm 3 ist um die Welle 7 drehbar und über das Lager 12 mit der Lagerungsplatte 1 fest verbunden, wobei ein Riemen oder eine Kette 19 zwischen den Zahnrädern 17 und 18, die eine Übersetzung 1:2 aufweisen, vorgesehen ist.

Die Befestigung der Zahnräder 14, 15, 17,18 an den erwähnten Elementen ist in der FIG. 4 mit jeweils zwei Schrauben dargestellt. Demnach ist das Zahnrad 14 mittels der Schrauben 20, 34 an dem Bodenstativ 5 fest angebracht und das Zahnrad 15 durch die Schrauben 21, 22 am Arm 3 befestigt. Das Zahnrad 17 ist mit Hilfe der Schrauben 23, 24 mit dem Arm 4 fest verbunden und das Zahnrad 18 am Lager 12 bzw. an der Lagerungsplatte 1 mittels der Schrauben 25,26 befestigt. Die Arme 3 und 4 sind über ein Kugellager 27 um die Welle 6 miteinander drehbar verbunden. Das andere Ende des Armes 3 ist über ein Kugellager 28 um die Welle 7 am Lager 12 angebracht und das andere Ende des Armes 4 ist über ein Kugellager 29 um die Welle 8 mit dem Bodenstativ 5 drehbar verbunden.

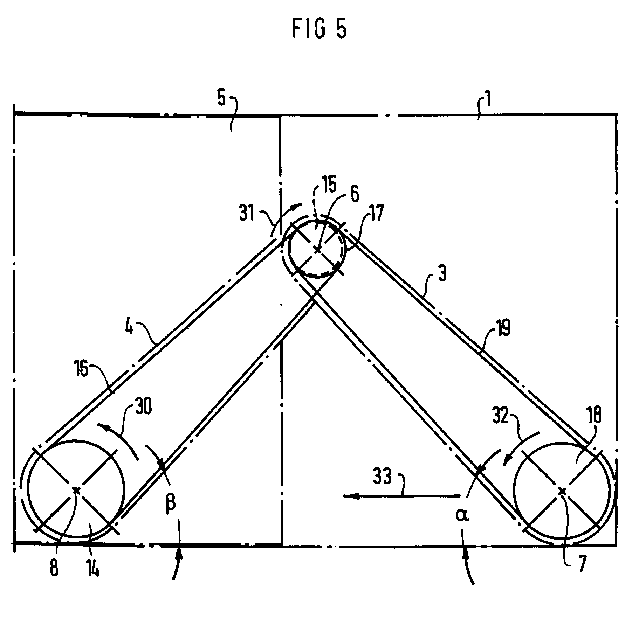

Die Steuerung des Gelenkarmes 2 bzw. der Lagerungsplatte 1 soll mit Hilfe der FIG. 5, die einen schematisch dargestellten Gelenkarm 2 nach FIG. 4 zeigt, näher beschrieben werden. Wenn der Arm 4 um die Welle 8 in Richtung des Pfeiles 30 gedreht wird, ist das Zahnrad 15, in dem das Zahnrad 14 am Bodenstativ fest angebracht ist, dazu gezwungen, auf den Riemen bzw. an der Kette zu klettern, so dass das Zahnrad 15 in die Richtung des Pfeiles 31 gedreht wird. Durch die bereits erwähnte Übersetzung zwischen den Zahnrädern 14 und 15 wird der Arm 3 durch seine feste Verbindung mit dem Zahnrad 15 doppelt soviel wie der Arm 4, aber in der entgegengesetzten Richtung, geschwenkt. Das Zahnrad 17 im Arm 3, das mit dem Arm 4 fest verbunden ist und daher den Bewegungen des Armes 4 folgt, zwingt nun das Zahnrad 18 auf dem Riemen oder auf die Kette 19 zu klettern, so dass das Zahnrad 18 in die Richtung des Pfeiles 32 gedreht wird. Durch die sukzessive Drehung des Zahnrades 18 im Verhältnis zur Bewegung des Armes 3 wird die Lagerungsplatte 1 in jeder Lage ausgerichtet, so dass die Lagerungsplatte 1 eine translatorische Bewegung in die Richtung des Pfeiles 33 beschreibt. Der Winkel zwischen der Längsseite der Lagerungsplatte 1 und dem Arm 4 ist dabei immer gleich dem Winkel zwischen der Längsseite der Lagerungsplatte 1 und dem Arm 3.

Die Bewegungen des riemen- oder kettentransmissiongetriebenen Gelenkarmes 2 können natürlich auch mittels eines Motors, der den Arm 4 um die Welle 8 antreibt, gesteuert werden. Die Lagerungsplatte 1 soll auch manuell verschoben werden können, indem der Benutzer die Lagerungsplatte 1 in eine gewünschte Lage verschiebt. Die Lagerungsplatte 1 kann dann auch in jeder Lage mit bekannten und daher nicht dargestellten Mitteln arretiert werden. Die Lagerungsplatte 1 kann auch derart freigekuppelt werden, dass sie gleichzeitig sowohl in die Längs- als auch in die Querrichtung bewegbar ist.

### Bezugszeichenliste

- 1: Lagerungsplatte
- 2: Gelenkarm
- 3: erster Arm
- 4: zweiter Arm
- 5: Bodenstativ
- 6: erste Welle, Verbindungsstelle
- 7: zweite Welle, Verbindungsstelle
- 8: dritte Welle, Verbindungsstelle
- 9,10,11: Motor
- α,β: Winkel
- 12: Lager
- 13: Schiene
- 14: erstes Zahnrad
- 15: zweites Zahnrad
- 16, 19: Riemen oder Kette, Transmission
- 17: drittes Zahnrad
- 18: viertes Zahnrad
- 20-26, 34: Schraube
- 27-29: Kugellager
- 30-33: Pfeil

## Patentansprüche

1. Untersuchungstisch für einen Patienten mit einer Lagerungsplatte (1) und einem Bodenstativ (5), wobei der Untersuchungstisch einen Gelenkarm (2) aufweist, der zwischen dem Bodenstativ (5) und der Lagerungsplatte (1) angeordnet ist und mit dem die Lagerungsplatte (1) gegenüber dem Bodenstativ (5) in der Längsrichtung der Lagerungsplatte (1) verschiebbar ist, **dadurch gekennzeichnet**, dass der Gelenkarm (2) mindestens zwei Arme (3,4) aufweist, deren eine Enden um eine erste Welle (6) miteinander drehbar verbunden sind, und daß das andere Ende des ersten Armes (3) mit der Lagerungsplatte (1) um eine zweite Welle (7) drehbar verbunden und das andere Ende des zweiten Armes (4) um eine dritte Welle (8) mit dem Bodenstativ (5) drehbar verbunden ist, wobei die Wellen (6, 7, 8) an den Verbindungsstellen gegenüber der horizontalen Ebene der Lagerungsplatte (1) vertikal angeordnet sind, so dass sich die Arme (3, 4) immer parallel zu dieser Ebene bewegen, wobei der Gelenkarm (2) mit Hilfe von Mitteln (9,10,11,14-19) derart steuerbar ist, dass der Winkel (α) zwischen der Längsseite der Lagerungsplatte (1) und dem ersten Arm (3) in allen Lagen gleich dem Winkel (β) zwischen der Längsseite der Lagerungsplatte (1) und dem zweiten Arm (4) ist.

2. Untersuchungstisch nach Anspruch 1, **dadurch gekennzeichnet**, dass die Arme (3, 4) des Gelenkarmes (2) in verschiedenen Ebenen angebracht sind.

3. Untersuchungstisch nach Anspruch 1 oder 2, **dadurch gekennzeichnet**, dass das andere Ende des ersten Armes (3) mit dem einen Ende der Lagerungsplatte (1), vorzugsweise mit dem Fussende, verbunden ist.

4. Untersuchungstisch nach einem der Ansprüche 1 bis 3, **dadurch gekennzeichnet**, dass das eine Ende der Lagerungsplatte (1) mit dem ersten Arm (3) derart verbunden ist, dass die Lagerungsplatte (1) gegenüber der Verbindungsstelle (7) senkrecht zur Längsrichtung der Lagerungsplatte (1) verschiebbar angeordnet ist.

5. Untersuchungstisch nach einem der Ansprüche 1 bis 4, **dadurch gekennzeichnet**, dass die Länge der Arme (3, 4) die grösste Breite der Lagerungsplatte (1) nicht überschreitet.

6. Untersuchungstisch nach einem der Ansprüche 1 bis 5, **dadurch gekennzeichnet**, dass die Lagerungsplatte (1) um die dritte Welle (8) an der Verbindungsstelle (8) am Bodenstativ (5) drehbar angeordnet ist.

7. Untersuchungstisch nach einem der Ansprüche 1 bis 6, **dadurch gekennzeichnet**, dass die Steuerung des Gelenkarmes (2) mittels dreier Motoren (9, 10, 11) erfolgt, bei denen der erste Motor (9) den zweiten Arm (4) um die dritte Welle (8) dreht, der zweite Motor (10) den ersten Arm (3) um die erste Welle (6) dreht, und der dritte Motor (11) die Lagerungsplatte (1) um die zweite Welle (7) dreht.

8. Untersuchungstisch nach einem der Ansprüche 1 bis 6, **dadurch gekennzeichnet**, dass der Gelenkarm (2) ein am Bodenstativ (5) fest angebrachtes erstes Zahnrad (14), das um die dritte Welle (8) angeordnet und im zweiten Arm (4) angebracht ist, und ein zweites Zahnrad (15), das im zweiten Arm (4) angeordnet ist, um die erste Welle (6) drehbar und mit dem ersten Arm (3) fest verbunden ist, sowie eine Transmission (16), die zwischen dem ersten und dem zweiten Zahnrad (14, 15) angeordnet ist, umfasst, wobei das erste und das zweite Zahnrad (14, 15) derart dimensioniert sind, dass eine Übersetzung 2:1 erreicht wird, wobei der Gelenkarm (2) ferner ein drittes Zahnrad (17), das im ersten Arm (3) angebracht und um die erste Welle (6) angeordnet und mit dem zweiten Arm (4) fest verbunden ist, und ein viertes Zahnrad (18), das im ersten Arm (3) appliziert und um die zweite Welle (7) drehbar und mit der Lagerungsplatte (1) fest verbunden ist, umfasst, wobei eine Transmission (19) zwischen dem dritten und dem vierten Zahnrad (17, 18) mit einer Übersetzung 1:2 eingestellt ist.

## Claims

1. Examining table for a patient, with a supporting panel (1) and a stand (5), the examining table having an articulated arm (2) which is arranged between the stand (5) and the supporting panel (1) and by means of which the supporting panel (1) can be displaced in its longitudinal direction with respect to the stand (5), characterized in that the articulated arm (2) has at least two arms (3, 4), one end of each of these arms being connected to one another such that they can rotate around a first shaft (6), and in that the other end of the first arm (3) is connected to the supporting panel (1) such that it can rotate around a second shaft (7) and the other end of the second arm (4) is connected to the stand (5) such that it can rotate around a third shaft (8), the shafts (6, 7, 8) at the connecting locations being arranged vertically with respect to the horizontal plane of the supporting panel (1), with the result that the arms (3, 4) always move parallel to this plane, it being possible for the articulated arm (3) to be controlled with the aid of means (9, 10, 11, 14-19) such that, in all positions, the angle (α) between the long side of the supporting panel (1) and the first arm (3) is equal to the angle (β) between the long side of the supporting panel (1) and the second arm (4).

2. Examining table according to Claim 1, characterized in that the arms (3, 4) of the articulated arm (2) are fitted in different planes.

3. Examining table according to Claim 1 or 2, characterized in that the other end of the first arm (3) is connected to one end of the supporting panel (1), preferably to the foot end.

4. Examining table according to one of Claims 1 to 3, characterized in that one end of the supporting panel (1) is connected to the first arm (3) such that the supporting panel (1) is arranged, in relation to the connecting location (7), such that it can be displaced perpendicularly with respect to the longitudinal direction of the supporting panel (1).

5. Examining table according to one of Claims 1 to 4, characterized in that the length of the arms (3, 4) does not exceed the largest width of the supporting panel (1).

6. Examining table according to one of Claims 1 to 5, characterized in that the supporting panel (1) is arranged on the stand (5) such that it can rotate around the third shaft (8) at the connecting location (8).

7. Examining table according to one of Claims 1 to 6, characterized in that the articulated arm (2) is controlled by means of three motors (9, 10, 11), of which the first motor (9) rotates the second arm (4) around the third shaft (8), the second motor (10) rotates the first arm (3) around the first shaft (6), and the third motor (11) rotates the supporting panel (1) around the second shaft (7).

8. Examining table according to one of Claims 1 to 6, characterized in that the articulated arm (2) comprises a first gear wheel (14), which is fixed on the stand (5) and is arranged around the third shaft (8) and is fitted in the second arm (4), and a second gear wheel (15), which is arranged in the second arm (4) and can be rotated around the first shaft (6) and is fixedly connected to the first arm (3), and a transmission means (16), which is arranged between the first and the second gear wheels (14, 15), the first and the second gear wheels (14, 15) being dimensioned so as to achieve a transmission ratio 2:1, and the articulated arm (2) further comprising a third gear wheel (17), which is fitted in the first arm (3) and is arranged around the first shaft (6) and is fixedly connected to the second arm (4), and a fourth gear wheel (18), which is provided in the first arm (3) and can be rotated around the second shaft (7) and is fixedly connected to the supporting panel (1), a transmission means (19) being set between the third and the fourth gear wheels (17, 18) with a transmission ratio 1:2.

## Revendications

1. Table d'examen destinée à un patient comportant un plateau de support (1) et un socle (5), la table d'examen étant munie d'un bras articulé (2) qui est disposé entre le socle (5) et le plateau de support (1), et par lequel le plateau de support (1) peut être déplacé par rapport au socle (5) dans la direction longitudinale du plateau de support (1), caractérisée par le fait que le bras articulé (2) comporte au moins deux bras (3, 4), qui par l'une de leurs extrémités sont reliés entre eux de façon à pouvoir pivoter autour d'un premier arbre (6), que l'autre extrémité du premier bras (3) est reliée au plateau de support (1) de façon à pouvoir pivoter autour d'un deuxième arbre (7), et que l'autre extrémité du deuxième bras (4) est reliée au socle (5) de façon à pouvoir pivoter autour d'un troisième arbre (8), les arbres (6, 7, 8) étant disposés aux points de liaison verticalement par rapport au plan horizontal du plateau de support (1), de sorte que les bras (3, 4) se déplacent toujours parallèlement à ce plan, le bras articulé (2) pouvant être commandé à l'aide de moyens (9, 10, 11, 14 à 19) de telle sorte que l'angle (α) entre le côté longitudinal du plateau de support (1) et le premier bras (3) soit dans toutes les positions égal à l'angle (β) entre le côté longitudinal du plateau de support (1) et le deuxième bras (4).

2. Table d'examen selon la revendication 1, caractérisée par le fait que les bras (3, 4) du bras articulé (2) sont disposés dans différents plans.

3. Table d'examen selon la revendication 1 ou 2, caractérisée par le fait que l'autre extrémité du premier bras (3) est reliée à l'une des extrémités du plateau de support (1), de préférence à l'extrémité située du côté pied.

4. Table d'examen selon l'une quelconque des revendications 1 à 3, caractérisée par le fait que l'une des extrémités du plateau de support (1) est reliée au premier bras (3) de telle sorte que, par rapport au point de liaison (7), le plateau de support (1) peut être déplacé perpendiculairement à la direction longitudinale du plateau de support (1).

5. Table d'examen selon l'une quelconque des revendications 1 à 4, caractérisée par le fait que la longueur des bras (3, 4) ne dépasse pas la plus grande largeur du plateau de support (1).

6. Table d'examen selon l'une quelconque des revendications 1 à 5, caractérisée par le fait que le plateau de support (1) est disposé sur le socle (5) de façon à pouvoir tourner autour du troisième arbre (8) au niveau du point de liaison (8).

7. Table d'examen selon l'une quelconque des revendications 1 à 6, caractérisée le fait que la commande du bras articulé (2) est effectuée au moyen de trois moteurs (9, 10, 11), le premier moteur (9) faisant tourner le deuxième bras (4) autour du troisième arbre (8), le deuxième moteur (10) faisant tourner le premier bras (3) autour du premier arbre (6), et le troisième moteur (11) faisant tourner le plateau de support (1) autour du deuxième arbre (7).

8. Table d'examen selon l'une quelconque des revendications 1 à 6, caractérisée par le fait que le bras articulé (2) comporte une première roue dentée (14) fixée sur le socle (5), qui est disposée autour du troisième arbre (8) et montée dans le deuxième bras (4), et une deuxième roue dentée (15) qui est disposée dans le deuxième bras (4), qui peut tourner autour du premier arbre (6) et qui est solidaire du premier bras (3), ainsi qu'une transmission (16) qui est disposée entre les première et deuxième roues dentées (14, 15), les première et deuxième roues dentées (14, 15) étant dimensionnées de façon à obtenir une démultiplication de 2:1, le bras articulé (2) comportant en outre une troisième roue dentée (17) qui est montée dans le premier bras (3), disposée autour du premier arbre (6) et solidaire du deuxième bras (4), et une quatrième roue dentée (18) qui est montée dans le premier bras (3), disposée de façon à pouvoir tourner autour du deuxième arbre (7) et solidaire du plateau de support (1), une transmission (19) étant réglée avec une démultiplication de 1:2 entre les troisième et quatrième roues dentées (17, 18).
